(19) Europäisches Patentamt
European Patent Office
Office européen des brevets

(11) Numéro de publication : **0 056 201**
**B1**

# (12) FASCICULE DE BREVET EUROPÉEN

(45) Date de publication du fascicule du brevet : **07.08.85**

(51) Int. Cl.[4] : **C 07 D453/02**

(21) Numéro de dépôt : **81400261.4**

(22) Date de dépôt : **20.02.81**

## (54) Nouveau procédé de préparation du N,N-diméthyl aza-1 bicyclo 2,2,2 octyl-3-10 10H-phénothiazinesulfonamide-2.

(30) Priorité : **13.01.81 FR 8100442**

(43) Date de publication de la demande : **21.07.82 Bulletin 82/29**

(45) Mention de la délivrance du brevet : **07.08.85 Bulletin 85/32**

(84) Etats contractants désignés : **AT BE CH DE GB IT LI LU NL SE**

(56) Documents cités :
**DE-A- 2 009 555**
**DE-B- 1 047 202**
**DE-B- 1 246 742**
**FR-A- 2 318 638**
**US-A- 2 909 521**
**Le dossier contient des informations techniques présentées postérieurement au dépôt de la demande et ne figurant pas dans le présent fascicule.**

(73) Titulaire : **PHARMUKA LABORATOIRES**
**35 Quai du Moulin de Cage**
**F-92231 Gennevilliers (FR)**

(72) Inventeur : **Renault, Christian Louis Albert**
**10, Allée Réaumur**
**F-95150 Taverny (FR)**

(74) Mandataire : **Gaumont, Robert et al**
**RHONE-POULENC RECHERCHES Service Brevets Pharma 25, Quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 056 201 B1

**Description**

La présente invention a pour objet un nouveau procédé de préparation du N,N-diméthyl [aza-1 bicyclo [2,2,2] octyl-3]-10 10H-phénothiazinesulfonamide-2, de formule :

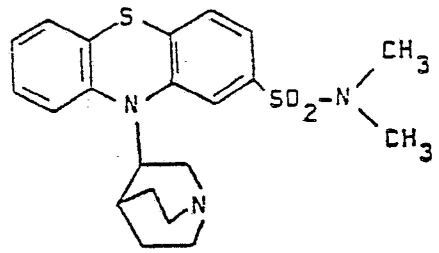

(I)

Le composé de formule (I) est un composé connu, utilisable notamment comme inhibiteur de la sécrétion gastrique, dont la préparation est décrite dans le certificat d'addition français 2 318 638.

Selon ce certificat d'addition, le composé de formule (I) est obtenu par condensation, au sein d'un solvant inerte constitué par un hydrocarbure aromatique et un solvant aprotique dipolaire, de l'ester benzènesulfonique de l'aza-1 bicyclo [2,2,2] octanol-3 de formule :

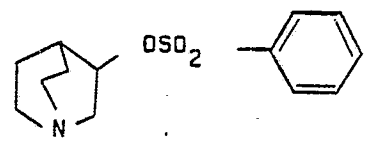

(II)

avec un dérivé métallique du N,N-diméthyl phénothiazine-sulfonamide-2 de formule :

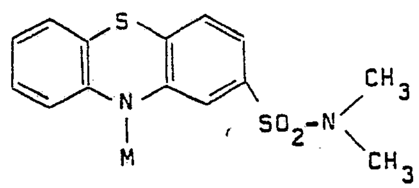

(III)

dans laquelle M désigne un métal alcalin, en particulier le sodium. Le rendement en composé de formule (I), calculé par rapport aux produits de départ (II) et (III), est de 40 %.

Le composé de formule (II) est lui-même préparé, en deux étapes, par réduction de l'aza-1 bicyclo [2,2,2] octanone-3 en aza-1 bicyclo [2,2,2] octanol-3 (cf C.A. GROB et coll., Helv. Chim. Acta, 1957, *40*, 2170) et action sur ce dernier du chlorure de benzènesulfonyle (cf E.E. MIKHLINA et coll., J. Gen. Chem. U.R.S.S., 1960, *30*, 2953). Le rendement en composé de formule (II), calculé par rapport à l'aza-1 bicyclo [2,2,2] octanone-3, est de 57,6 %.

Le composé de formule (III) est obtenu par action, au sein d'un solvant inerte tel qu'un hydrocarbure aromatique, d'un hydrure ou d'un amidure de métal alcalin sur le N,N-diméthyl phénothiazinesulfoamide-2 qui est lui-même obtenu, avec un rendement de 71 %, par cyclisation du N, N-diméthyl amino-3 [(bromo-2 phényl) thio]-4 benzènesulfonamide (cf V.V. SHAVYRINA et coll., Med. Prom. U.R.S.S., 1966, *20*, 15).

Au total, lorsqu'on utilise le procédé décrit dans le certificat d'addition français 2 318 638, il faut cinq étapes pour synthétiser le composé de formule (I) à partir de l'aza-1 bicyclo [2,2,2] octanone-3 et du N,N-diméthyl amino-3 [(bromo-2 phényl)thio]-4 benzènesulfonamide et le rendement en composé (I) n'est que de 23 % par rapport à l'aza-1 bicyclo [2,2,2] octanone-3 et de 28 % par rapport au N,N-diméthyl amino-3 [(bromo-2 phényl)thio]-4 benzènesulfonamide. La préparation de dérivés de phénothiazine par cyclisation de dérivés d'amino-2 halogéno-2' diphénylsulfure est décrite dans le brevet DE 1 047 202.

Il a maintenant été trouvé, conformément à la présente invention, un nouveau procédé de préparation du composé de formule (I), qui permet de synthétiser ce composé à partir de l'aza-1 bicyclo [2,2,2] octanone-3 et du N,N-diméthyl amino-3 [(bromo-2 phényl)thio]-4 benzènesulfonamide en trois étapes au lieu de cinq et d'augmenter notablement le rendement en composé (I) par rapport à l'aza-1 bicyclo [2,2,2] octanone-3 et au N,N-diméthyl amino-3 [(bromo-2 phényl)thio]-4 benzènesulfonamide.

Ce nouveau procédé consiste à effectuer la cyclisation du composé de formule (IV) ci-dessous, selon le schéma réactionnel :

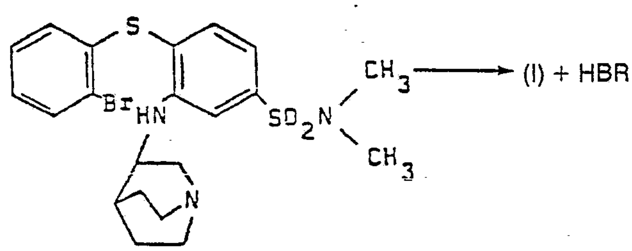


(IV)

La réaction de cyclisation est effectuée en présence d'un amidure de métal alcalin, en particulier l'amidure de sodium. Elle peut être effectuée soit au sein de l'ammoniac liquide, à une température comprise entre −40 °C et la température d'ébullition de l'ammoniac liquide sous la pression atmosphérique, soit au sein d'un solvant aprotique tel que la tétrahydrofuranne, en présence ou non d'un alcool, à une température comprise entre 20 °C et la température d'ébullition du solvant. Dans la variante en milieu solvant aprotique, on opère de préférence en présence d'un alcool tel que le tertiobutanol et à une température voisine de 20 °C.

Le composé de formule (IV) ou N,N-diméthyl [(aza-1 bicyclo [2,2,2] octyl-3)amino]-3 [(bromo-2 phényl)thio]-4 benzènesulfonamide peut être préparé par condensation de l'aza-1 bicyclo [2,2,2] octanone-3 avec le N,N-diméthyl amino-3 [(bromo-2 phényl)thio]-4 benzènesulfonamide de formule (V) selon le schéma réactionnel suivant :

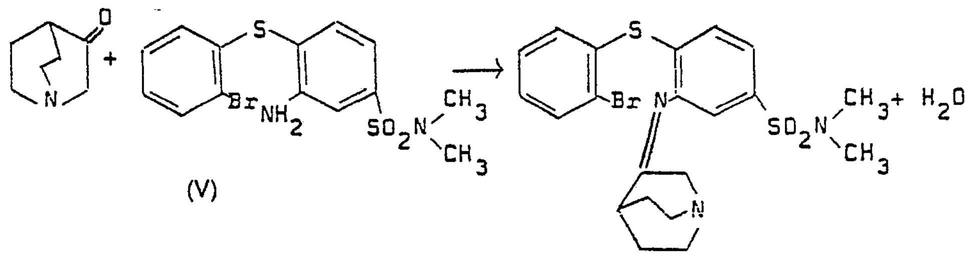

(V)

(VI)

et réduction de l'imine de formule (VI) ainsi formée.

Pour la réaction de condensation on utilise l'aza-1 bicyclo [2,2,2] octanone-3 à l'état de base libre ou d'un sel de la base (chlorhydrate par exemple). La réaction de condensation est effectuée au sein d'un hydrocarbure aromatique tel que le xylène, en présence d'un acide protonique (par exemple l'acide p-toluènesulfonique) et à la température d'ébullition du milieu (ce qui permet d'éliminer l'eau formée par distillation azéotropique), soit au sein d'un solvant inerte tel que le chloroforme, en présence d'un agent de déshydratation (par exemple le tétrachlorure de titane) et à une température comprise entre − 5 °C et +10 °C.

La réduction de l'imine de formule (VI) peut être effectuée au moyen d'un borohydrure de métal alcalin (par exemple le borohydrure de sodium), au sein d'un alcool tel que l'éthanol et à une température comprise entre 0 °C et 40 °C. Pour effectuer la réaction de réduction il n'est pas nécessaire d'isoler à l'état pur l'imine (VI) formée dans la réaction de condensation précédente.

Le composé de formule (I) formé par cyclisation du composé de formule (IV) est isolé à l'état pur à partir des mélanges réactionnels par des méthodes classiques, physiques (évaporation, extraction à l'aide d'un solvant, distillation, cristallisation ou chromatographie) ou chimiques (formation de sel et régénération de la base).

Les exemples suivants illustrent l'invention.

## Exemple 1

Préparation du N,N-diméthyl [(aza-1 bicyclo [2,2,2] octyl-3)amino]-3 [(bromo-2 phényl)thio]-4 benzènesulfonamide.

Dans 150 ml de chloroforme, placés sous atmosphère d'azote sec, on introduit 4,85 g de chlorhydrate d'aza-1 bicyclo [2,2,2] octanone-3, 11,61 g de N,N-diméthyl amino-3 [(bromo-2 phényl)thio]-4 benzènesulfonamide et 25 ml de triéthylamine. On agite et refroidit la solution obtenue à − 5 °C, toujours en agitant. On ajoute, en l'espace de 90 minutes, une solution de 3,3 ml de tétrachlorure de titane dans 15 ml de chloroforme. On continue à agiter à −5 °C pendant 20 heures puis, en maintenant la température inférieure à 0 °C, on ajoute 10 ml d'éthanol absolu.

Le chloroforme est évaporé sous pression réduite et le résidu est dissous dans 150 ml d'éthanol absolu. On ajoute 7,5 g d'hydroxyde de sodium en pastilles, puis 2,3 g de borohydrure de sodium. On agite 20 heures à la température ambiante. On filtre, lave l'insoluble avec 4 fois 50 ml d'éthanol absolu, réunit le filtrat et les solutions de lavage et les évapore sous pression réduite.

Le résidu est repris par 50 ml d'eau et 50 ml de toluène. La phase toluène est séparée par décantation et la phase aqueuse est extraite par 2 fois 50 ml de toluène. Les phases toluène rassemblées sont extraites par 50 ml d'une solution aqueuse N d'acide méthanesulfonique et 4 fois 25 ml d'eau. La phase aqueuse acide est lavée avec deux fois 50 ml d'oxyde de diéthyle, amenée à pH ~ 8 par addition d'une solution concentrée d'ammoniaque et l'huile qui relargue est extraite avec 4 fois 50 ml de toluène. Les phases toluène sont séchées sur du sulfate de magnésium anhydre, filtrées et évaporées sous pression réduite. On obtient ainsi 10,4 g de N,N-diméthyl [(aza-1 bicyclo [2,2,2] octyl-3) amino]-3 [(bromo-2 phényl)thio]-4 benzènesulfonamide, qui fond à 129-130 °C. Le rendement en ce dernier produit, calculé par rapport à l'aza-1 bicyclo [2,2,2] octanone-3 de départ, est d'environ 70 %.

Exemple 2

Préparation du N,N-diméthyl [(aza-1 bicyclo [2,2,2] octyl-3)amino]-3 [(bromo-2 phényl)thio]-4 benzènesulfonamide.

On chauffe pendant 50 heures au reflux, en éliminant l'eau qui se forme par distillation azéotropique, un mélange de 70 ml de xylène, 3,87 g de N,N-diméthyl amino-3 [(bromo-2 phényl)thio]-4 benzènesulfonamide, 1,25 g d'aza-1 bicyclo [2,2,2] octanone-3 et 0,19 g d'acide p-toluènesulfonique.

On élimine la majeure partie du solvant par évaporation sous pression réduite, ajoute 25 ml d'éthanol, 0,5 g d'hydroxyde de sodium et 0,76 g de borohydrure de sodium. Après une heure d'agitation à la température ambiante, on ajoute 50 ml d'eau, amène le pH à 1 par addition d'une solution concentrée d'acide chlorhydrique et évapore l'éthanol sous pression réduite. La phase aqueuse est alcalinisée par addition d'hydroxyde de potassium et l'insoluble est extrait avec 100 ml puis deux fois 50 ml de toluène. Les phases toluène sont lavées avec 25 ml d'eau, puis extraites avec 50 ml d'une solution aqueuse N d'acide méthanesulfonique et 5 fois 25 ml d'eau. La phase aqueuse acide est alcalinisée par addition d'une solution concentrée d'ammoniaque et l'huile qui relargue est extraite avec deux fois 50 ml puis 3 fois 25 ml de chloroforme. Les phases chloroformiques sont séchées sur du sulfate de magnésium et évaporées sous pression réduite. On obtient ainsi 3,57 g de N,N-diméthyl [(aza-1 bicyclo [2,2,2] octyl-3) amino]-3 [(bromo-2 phényl)thio]-4 benzènesulfonamide. Le rendement, calculé par rapport à l'aza-1 bicyclo [2,2,2] octanone-3 ou au N,N-diméthyl amino-3 [(bromo-2 phényl)thio]-4 benzènesulfonamide, est de 72 %.

Exemple 3

Préparation du N,N-diméthyl [aza-1 bicyclo [2,2,2] octyl-3]-10 10H-phénothiazinesulfonamide-2.

Dans 10 ml de tétrahydrofuranne anhydre, placés sous atmosphère d'azote sec, on introduit, à la température ambiante, 1,91 g d'amidure de sodium en poudre. On ajoute 1,03 g d'alcool tert-butylique et 5 ml de tétrahydrofuranne anhydre. On chauffe la suspension à 45 °C pendant deux heures, puis on laisse revenir à la température ambiante. On ajoute alors, sous agitation, une solution de 3,47 g de N,N-diméthyl [(aza-1 bicyclo [2,2,2] octyl-3)amino]-3 [(bromo-2 phényl)thio]-4 benzènesulfonamide dans 20 ml de tétrahydrofuranne et continue l'agitation pendant 24 heures à la température ambiante.

En refroidissant, on coule 10 ml d'eau dans le milieu réactionnel et verse le mélange obtenu dans 50 ml d'eau et 50 ml de toluène. La phase toluène est séparée et la phase aqueuse est extraite par deux fois 25 ml de toluène. Les phases toluène rassemblées sont extraites par 25 ml d'une solution aqueuse normale d'acide méthanesulfonique, puis par 4 fois 25 ml d'eau. Les phases aqueuses rassemblées sont lavées avec deux fois 25 ml d'éther, amenées à pH 8 par addition d'une solution concentrée d'ammoniaque et l'insoluble est extrait avec 4 fois 50 ml d'acétate d'éthyle. La phase organique est séchée sur du sulfate de magnésium, filtrée et évaporée sous pression réduite. Le résidu obtenu (1,51 g) est fixé sur une colonne de gel de silice et on élue avec un mélange de 95 parties en volume de toluène et 5 parties en volume de diéthylamine. On récupère ainsi 1,41 g de produit que l'on recristallise dans 10 ml d'un mélange d'une partie en volume d'oxyde de diéthyle et d'une partie en volume de butanone-2. La recristallisation fournit 0,95 g de N,N-diméthyl [aza-1 bicyclo [2,2,2] octyl-3]-10 10H-phénothiazinesulfonamide-2. Ce produit présente un point de fusion commençante de 135 °C, un point de fusion franche de 156 °C et se resolidifie lentement pour refondre à 183 °C (mesures effectuées au banc Köfler).

Exemple 4

Préparation du N,N-diméthyl [aza-1 bicyclo [2,2,2] octyl-3]-10 10H-phénothiazinesulfonamide-2.

A 600 ml d'ammoniac liquide, maintenu à sa température d'ébullition sous la pression atmosphérique (environ − 30 °C), on ajoute 2,3 g de sodium et un cristal de nitrate ferrique. Après une heure d'agitation, la solution bleu foncé a fait place à une suspension grise. On refroidit à − 40 °C et ajoute rapidement, en agitant, une solution de 12,4 g de N,N-diméthyl [(aza-1 bicyclo [2,2,2] octyl-3) amino]-3 [(bromo-2 phényl)thio]-4 benzènesulfonamide dans 60 ml de tétrahydrofuranne anhydre. On poursuit l'agitation pendant 1 heure et 15 minutes à la température d'ébullition de l'ammoniac liquide, puis on ajoute 20 g de chlorure d'ammonium et laisse l'ammoniac s'évaporer.

Le résidu est repris par 100 ml de toluène et 100 ml d'eau. On agite, sépare la phase toluène par décantation et extrait la phase aqueuse avec 2 fois 50 ml de toluène. Les phases toluène rassemblées sont estraites avec 100 ml d'une solution aqueuse N d'acide méthanesulfonique puis avec 5 fois 50 ml d'eau. Les phases aqueuses rassemblées sont lavées avec 2 fois 100 ml d'oxyde de diéthyle, amenées à pH 8 par addition d'une solution concentrée d'ammoniaque et l'insoluble est extrait avec 4 fois 100 ml d'acétate d'éthyle. La phase organique est séchée sur du sulfate de magnésium anhydre, filtrée et évaporée sous pression réduite. Le résidu obtenu (9,57 g) est fixé sur une colonne de gel de silice et on élue avec un mélange toluène-diéthylamine 90/10. On récupère ainsi 7,4 g de produit que l'on recristallise dans 20 ml

d'acétone. La recristallisation fournit 6,46 g de N,N-diméthyl [aza-1 bicyclo [2,2,2] octyl-3]-10 10H-phénothiazinesulfonamide-2. Ce produit présente les mêmes points de fusion que celui obtenu à l'exemple 3.

Le rendement en N,N-diméthyl [aza-1 bicyclo [2,2,2] octyl-3]-10 10H-phénothiazinesulfonamide-2, calculé par rapport à l'aza-1 bicyclo [2,2,2] octanone-3 ou au N,N-diméthyl amino-3 [(bromo-2 phényl)thio]-4 benzènesulfonamide, est de 44 %.

**Revendications**

1. Procédé de préparation du composé de formule :

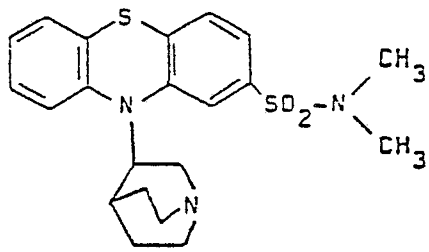

(I)

caractérisé en ce qu'on réalise la cyclisation du composé de formule :

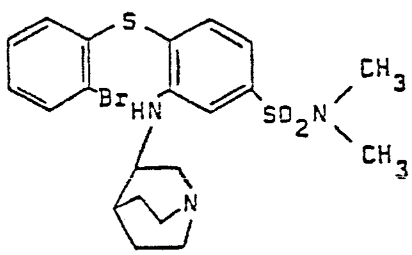

(IV)

en présence d'un amidure de métal alcalin soit dans l'ammoniac liquide, à une température comprise entre − 40 °C et la température d'ébullition de l'ammoniac liquide sous la pression atmosphérique, soit dans un solvant aprotique, en présence ou non d'un alcool, à une température comprise entre 20 °C et la température d'ébullition du solvant.

2. Procédé selon la revendication 1, caractérisé en ce que l'on opère en présence d'un alcool et à une température voisine de 20 °C.

3. Procédé selon l'une quelconque des revendications 1 ou 2 caractérisé en ce que le composé de formule (IV) est obtenu par condensation de l'aza-1 bicyclo [2,2,2] octanone-3 avec le N,N-diméthyl amino-3 [(bromo-2 phényl)thio]-4 benzènesulfonamide et réduction de l'imine de formule :

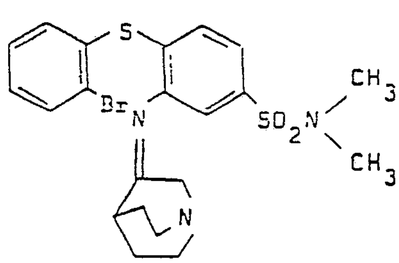

(VI)

ainsi obtenue.

4. Procédé selon la revendication 3, caractérisé en ce que la condensation de l'aza-1 bicyclo [2,2,2] octanone-3 avec le N,N-diméthyl amino-3 [(bromo-2 phényl)thio]-4 benzènesulfonamide est effectuée au sein d'un hydrocarbure aromatique, en présence d'un acide protonique et à la température d'ébullition du milieu.

5. Procédé selon la revendication 3, caractérisé en ce que la condensation de l'aza-1 bicyclo [2,2,2] octanone-3 avec le N,N-diméthyl amino-3 [(bromo-2 phényl)thio]-4 benzènesulfonamide est effectuée au sein d'un solvant inerte, en présence d'un agent de déshydratation et à une température comprise entre − 5 °C et +10 °C.

**Claims**

1. Process for preparing the compound of formula :

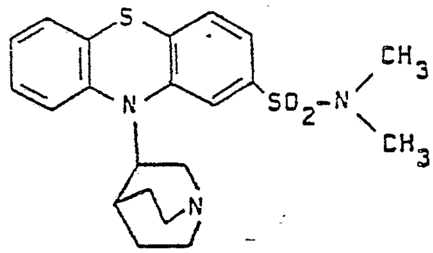

(I)

characterised in that cyclisation of the compound of formula :

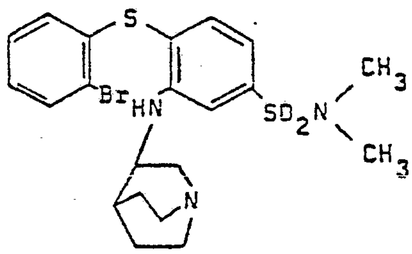

(IV)

is carried out in the presence of an alkali metal amide either in liquid ammonia, at a temperature between − 40 °C and the boiling point of liquid ammonia at atmospheric pressure, or in an aprotic solvent, in the presence or absence of an alcohol, at a temperature between 20 °C and the boiling point of the solvent.

2. Process according to Claim 1, characterised in that the operation is carried out in the presence of an alcohol and at a temperature in the region of 20 °C.

3. Process according to either of Claims 1 or 2, characterised in that the compound of formula (IV) is obtained by condensing 1-azabicyclo [2,2,2]-3-octanone with N,N-dimethyl-3-amino-4-[(2-bromophenyl)-thio] benzenesulphonamide and reduction of the imine of formula :

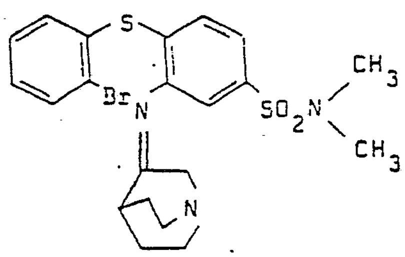

(VI)

thus obtained.

4. Process according to Claim 3, characterised in that the condensation of 1-azabicyclo [2,2,2]-3-octanone with N,N-dimethyl-3-amino-4-[(2-bromophenyl)thio] benzenesulphonamide is carried out in an aromatic hydrocarbon, in the presence of a protonic acid and at the boiling point of the medium.

5. Process according to Claim 3, characterised in that the condensation of 1-azabicyclo [2,2,2]-3-octanone with N,N-dimethyl-3-amino-4-[(2-bromophenyl)thio] benzenesulphonamide is carried out in an inert solvent, in the presence of a dehydrating agent and at a temperature between − 5 °C and +10 °C.

**Patentansprüche**

1. Verfahren zur Herstellung der Verbindung der Formel I :

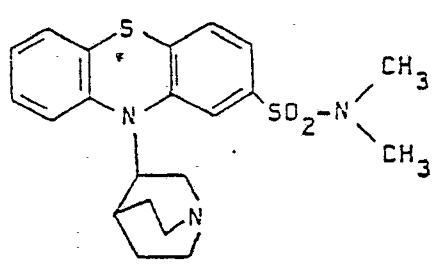

(I)

dadurch gekennzeichnet, daß man die Cyclisierung der Verbindung der Formel IV :

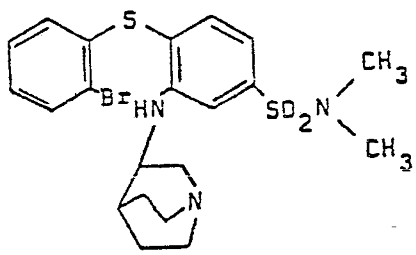

(IV)

in Gegenwart eines Alkalimetallamids entweder in flüssigem Ammoniak bei einer Temperatur zwischen − 40 °C und der Siedetemperatur des flüssigen Ammoniaks unter Atmosphärendruck oder in einem aprotischen Lösungsmittel in Gegenwart oder nicht eines Alkohols bei einer Temperatur zwischen 20 °C und der Siedetemperatur des Lösungsmittels bewirkt.

2. Verfahren gemäß Anspruch 1, dadurch gekennzeichnet, daß man in Gegenwart eines Alkohols und bei einer Temperatur von etwa 20 °C arbeitet.

3. Verfahren gemäß einem der Ansprüche 1 oder 2, dadurch gekennzeichnet, daß die Verbindung der Formel IV erhalten ist durch Kondensation von 1-Aza-bicyclo [2,2,2] octanon-3 mit N,N-Dimethyl-3-amino-4-[(2-bromphenyl)-thio]-benzolsulfonamid und Reduktion des so erhaltenen Imins der formel VI :

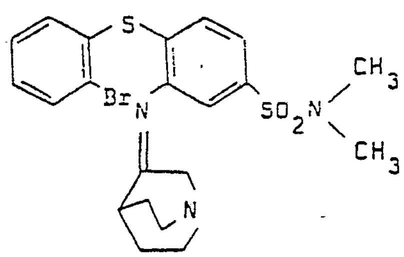

(VI)

4. Verfahren gemäß Anspruch 3, dadurch gekennzeichnet, daß die Kondensation des 1-Aza-bicyclo [2,2,2] octanon-3 mit dem -N,N-Dimethyl-3-amino-4-[(2-bromphenyl)-thio]-benzolsulfonamid in einem aromatischen Kohlenwasserstoff in Gegenwart einer Protonensäure und bei der Siedetemperatur des Milieus durchgeführt wird.

5. Verfahren gemäß Anspruch 3, dadurch gekennzeichnet, daß die Kondensation des 1-Aza-bicyclo [2,2,2] octanon-3 mit dem -N,N-Dimethyl-3- amino-4[(2-bromphenyl)-thio]-benzolsulfonamid in einem inerten Lösungsmittel in Gegenwart eines Dehydratationsmittels und bei einer Temperatur zwischen − 5 °C und + 10 °C durchgeführt wird.